# EUROPEAN PATENT APPLICATION

(11) **EP 4 285 814 A1**
(43) Date of publication of application: **06.12.2023**
(21) Application number: 23175480.5
(22) Date of filing: 25.05.2023
(51) Int. Cl.: A61B 5/00

(54) **IMPLANTABLE CARDIAC MONITOR**

(30) Priority: 25.05.2022 US 202263345623 P
(71) Applicant: Li, Bing, Toledo, OH 43606 (US)
(72) Inventor: Li, Bing, Toledo, OH 43606 (US)
(74) Representative: D Young & Co LLP

(57) **Abstract**

An implantable cardiac monitor and associated methods are described. The implantable cardiac monitor comprises a main body having a first electrode and a second electrode, the first electrode being in electrical communication with the second electrode, and the first electrode and the second electrode being adapted to receive cardiac electrical signals from a patient relative to a first position; and a tail member connected to the main body and having a third electrode and a fourth electrode, the third electrode being in electrical communication with the fourth electrode, and the third electrode and the fourth electrode being adapted to receive cardiac electrical signals from the patient relative to a second position.

## Description

### RELATED APPLICATIONS

This application claims priority to United States Provisional Application No. 63/345,623, filed under 35 U.S.C. § 111(b) on May 25, 2022. The entire disclosure of the aforementioned application is expressly incorporated hereby by reference for all purposes.

### BACKGROUND

Most of current implantable cardiac monitors are prone to problems or otherwise have disadvantages such as being too complicated, being unable to detect acute coronary syndrome events, having a high error rate when detecting event of atrial fibrillation, ventricular tachycardia, or other cardiac events, and having poor battery life. Thus, there is room in the art for new and improved implantable cardiac monitors, systems, and related methods.

### SUMMARY

Particular aspects are set out in the appended independent claims. Various optional embodiments are set out in the dependent claims.

Provided herein an example implantable cardiac monitor comprising a main body having a first electrode and a second electrode, the first electrode being in electrical communication with the second electrode, and the first electrode and the second electrode being adapted to receive electrical signals relative to a first position; and a tail member connected to the main body and having a third electrode and a fourth electrode, the third electrode being in electrical communication with the fourth electrode, and the third electrode and the fourth electrode being adapted to receive electrical signals relative to a second position.

In certain embodiments, the main body further comprises a motherboard including a processor and a memory, the memory including a computer readable medium with processor-executable instructions stored thereon.

In certain embodiments, the motherboard includes a monitoring module configured to generate monitoring data based on based on the received electrical signals, and the memory is configured to store the monitoring data.

In certain embodiments, the monitoring data includes at least one a heartrate, a P-wave, a PR interval, a PR segment, a QRS complex, a ST segment, a T wave, or a combination thereof.

In certain embodiments, the monitoring module is configured to predict a cardiac event based on the monitoring data.

In certain embodiments, the cardiac event includes at least one of atrial fibrillation, atrial flutter, ventricular fibrillation, ventricular tachycardia, pause, atrial brady, atrial tachycardia, atrial bigeminy, atrial trigeminy, ventricular bigeminy, ventricular trigeminy, acute coronary syndrome, or a combination thereof.

In certain embodiments, the monitoring module includes an algorithm configured to predict the cardiac event.

In certain embodiments, the main body further comprises an internal battery in electrical communication with the motherboard.

In certain embodiments, the internal battery has a rectangular shape with rounded corners.

In certain embodiments, the main body further comprises an internal coil in electrical communication with the internal battery and configured to be inductively coupled to an external coil. When the implantable cardiac monitor is implanted in the body of a patient, the external coil can be outside the body of the patient.

In certain embodiments, the main body further comprises an internal antenna in electrical communication with the motherboard and configured to be in wireless communication with an external monitoring device.

In certain embodiments, the implantable cardiac monitor further comprises a second tail member having one or more electrodes being adapted to receive electrical signals relative to a third position.

In certain embodiments, the implantable cardiac monitor further comprises a third tail member having one or more electrodes being adapted to receive electrical signals relative to a fourth position.

In certain embodiments, the implantable cardiac monitor further comprises a fourth tail member having one or more electrodes being adapted to receive electrical signals relative to a fifth position.

Further provided herein is a cardiac monitoring system comprising an implantable cardiac monitor comprising a main body having a first electrode, a second electrode, and an internal coil, the first electrode being in electrical communication with the second electrode, and the first electrode and the second electrode being adapted to receive cardiac electrical signals relative to a first position; and a tail member connected to the main body and having a third electrode and a fourth electrode, the third electrode being in electrical communication with the fourth electrode, and the third electrode and the fourth electrode being adapted to receive electrical signals relative to a second position; and an external charging device having an external coil configured to be inductively coupled to the internal coil.

Further provided herein is a cardiac monitoring system comprising an implantable cardiac monitor comprising a main body having a first electrode, a second electrode, and an internal antenna, the first electrode in electrical communication with the second electrode, and the first electrode and the second electrode adapted to receive cardiac electrical signals from a patient relative to a first position; and a tail member connected to the main body and having a third electrode and a fourth electrode, the third electrode in electrical communication with the fourth electrode, and the third electrode and the fourth electrode being adapted to receive electrical signals relative to a second position; and an external monitoring device configured to be in communication with the internal antenna and receiving monitoring data based on received electrical signals relative to the first position and relative to the second position.

In certain embodiments, the external monitoring device is a smart watch.

In certain embodiments, the external monitoring device is configured to provide an alert based on the monitoring data.

In certain embodiments, the cardiac monitoring system comprises a server configured to be in communication with at least one of the implantable cardiac monitor, the external monitoring device, or a combination thereof and receive the monitoring data.

In certain embodiments, the server is configured to provide an alert or the monitoring data to medical service providers.

Further provided is a method of performing a medical procedure comprising advancing the main body of the implantable cardiac monitor under the skin of a patient; and advancing the tail member of the implantable cardiac monitor under the skin of the patient; wherein the main body extends into a first position and the tail member extends into a second position, the first position is oriented away from one side of a heart of the patient and the second position is oriented away from another side of the heart of the patient.

Further provided is a method of monitoring cardiac electrical signals of a patient, the method comprising detecting cardiac electrical signals with an implantable cardiac monitor, generating monitoring data based on the detected cardiac electrical signals, storing the monitoring data into a memory of the implantable cardiac monitor, transferring the monitoring data from the implantable cardiac monitor to an external monitoring device, transferring the monitoring data from the external monitoring device to a server, and contacting, through the server, medical service providers based on the monitoring data.

In certain embodiments, the method further includes a step of advancing a second tail member into a third position and extending the second tail member into a third direction.

In certain embodiments, the method further includes a step of advancing a third tail member into a fourth position and extending the third tail member into a fourth direction.

In certain embodiments, the method further includes a step of advancing a fourth tail member into a fifth position and extending the fourth tail member into a fifth direction.

Further provided is an implantable cardiac monitor comprising a main body having a motherboard including a processor and a memory, the memory including a computer readable medium comprising processor-executable instructions; and a tail member connected to the main body and having one or more electrodes being adapted to receive electrical signals relative to a first position.

In certain embodiments, the motherboard includes a monitoring module configured to generate monitoring data based on received cardiac electrical signals from a patient, and the memory is configured to store the monitoring data.

In certain embodiments, the monitoring data includes at least one a heartrate, a P-wave, a PR interval, a PR segment, a QRS complex, a ST segment, a T wave, or a combination thereof.

In certain embodiments, the monitoring module is configured to predict a cardiac event based on the monitoring data.

In certain embodiments, the cardiac event includes at least one of atrial fibrillation, atrial flutter, ventricular fibrillation, ventricular tachycardia, pause, atrial brady, atrial tachycardia, atrial bigeminy, atrial trigeminy, ventricular bigeminy, ventricular trigeminy, acute coronary syndrome, or a combination thereof.

In certain embodiments, the monitoring module includes an algorithm configured to predict the cardiac event.

In certain embodiments, the main body further comprises an internal battery in electrical communication with the motherboard.

In certain embodiments, the internal battery has a rectangular shape with rounded corners.

In certain embodiments, the main body further comprises an internal coil in electrical communication with the internal battery and configured to be inductively coupled to an external coil.

In certain embodiments, the main body further comprises an internal antenna in electrical communication with the motherboard and configured to be in wireless communication with an external monitoring device.

In certain embodiments, the implantable cardiac monitor further comprises a second tail member having one or more electrodes being adapted to receive electrical signals relative to a second position.

In certain embodiments, the implantable cardiac monitor further comprises a third tail member having one or more electrodes being adapted to receive electrical signals relative to a third position.

In certain embodiments, the implantable cardiac monitor further comprises a fourth tail member having one or more electrodes being adapted to receive electrical signals relative to a fourth position.

In certain embodiments, the main body includes one or more electrodes being adapted to receive electrical signals relative to a fifth position.

Various aspects of the present teachings will become apparent to those skilled in the art from the following detailed description of the illustrative embodiments, when read in light of the accompanying drawings.

### BRIEF DESCRIPTION OF THE DRAWINGS

The patent or application file may contain one or more drawings executed in color and/or one or more photographs.
**FIG. 1** is top plan view of a non-limiting example implantable cardiac monitor.
**FIG. 2** is cross sectional view of a non-limiting example implantable cardiac monitor.
**FIG. 3** is a top plan view of a non-limiting example implantable cardiac monitor, including a tail member.
**FIG. 4** shows a non-limiting example cardiac monitoring system which includes the implantable cardiac monitor shown in **FIG. 3** and an electric charging device.
**FIG. 5** shows various views of another non-limiting example cardiac monitoring system which includes the implantable cardiac monitor shown in **FIG. 3****,** an external monitoring device, and a server.
**FIG. 6** is a front elevational view of an example placement of the main body (shown as line A-A) and the tail member (shown as line B-B) of an implantable cardiac monitor as described herein in proximity to a heart, such as in the body of a patient.
**FIG. 7** is a flowchart illustrating the implantable cardiac monitor communicating with an external device and a server.
**FIG. 8** is an example of a standard electrocardiogram.
**FIG. 9** is a top plan view of a non-limiting example implantable cardiac monitor having one tail member.
**FIG. 10** is a top plan view of a non-limiting example implantable cardiac monitor having two tail members.
**FIG. 11** is a top plan view of a non-limiting example implantable cardiac monitor having three tail members.
**FIG. 12** is a top plan view of a non-limiting example implantable cardiac monitor having four tail members.
**FIG. 13** is a front elevational view of another non-limiting example placement of the main body (shown as line A-A) and the tail member (shown as line B-B) of an implantable cardiac monitor as described herein in proximity to a heart, such as in the body of a patient.

### DETAILED DESCRIPTION

The present disclosure relates to improved implantable cardiac monitors, systems, and associated methods.

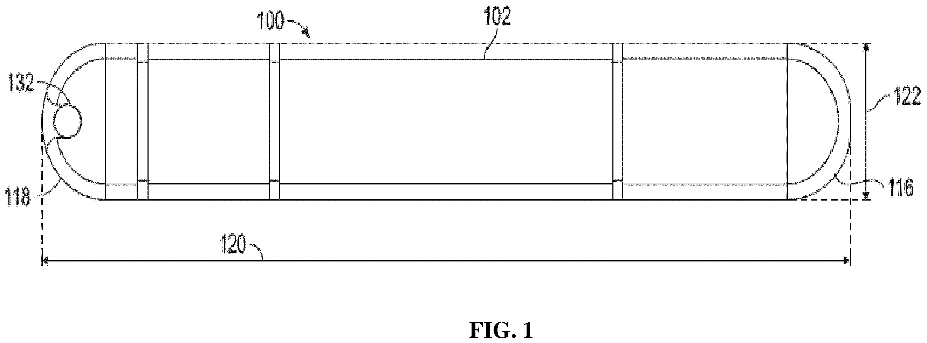

Referring now to the drawings, there are examples of an implantable cardiac monitor 100 illustrated in **FIGS. 1-3** and **FIGS. 9-12****.** The implantable cardiac monitor 100 may include a main body 102 and one or more tail members 104, e.g., a first tail member 104a, a second tail member 104b, a third tail member 104c, and a fourth tail member 104d. As will be described below, the implantable cardiac monitor 100 can be implanted under the skin of a patient to monitor cardiac electrical signals of the patient, or may otherwise be disposed in proximity to a heart to monitor cardiac electrical signals from the heart. As used herein the term "skin" can include an epidermis layer, a dermis layer, a hypodermis layer (subcutaneous tissue), or combinations thereof.

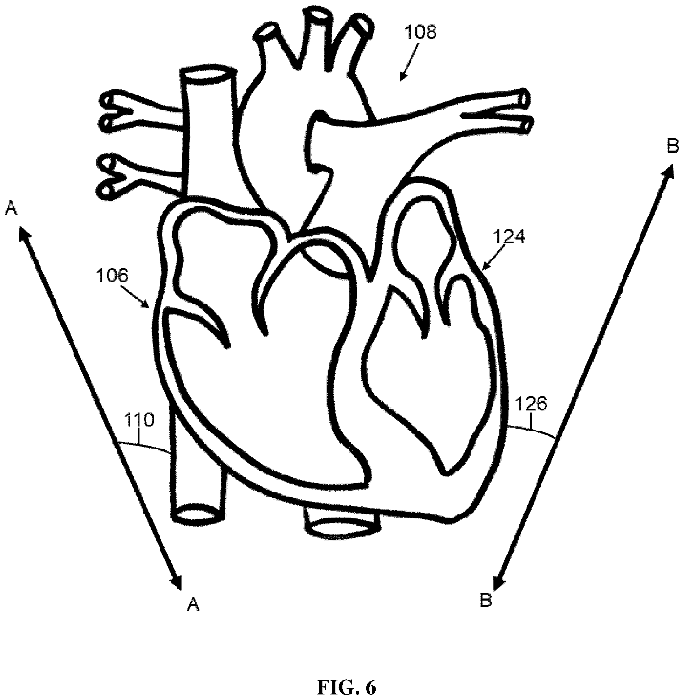

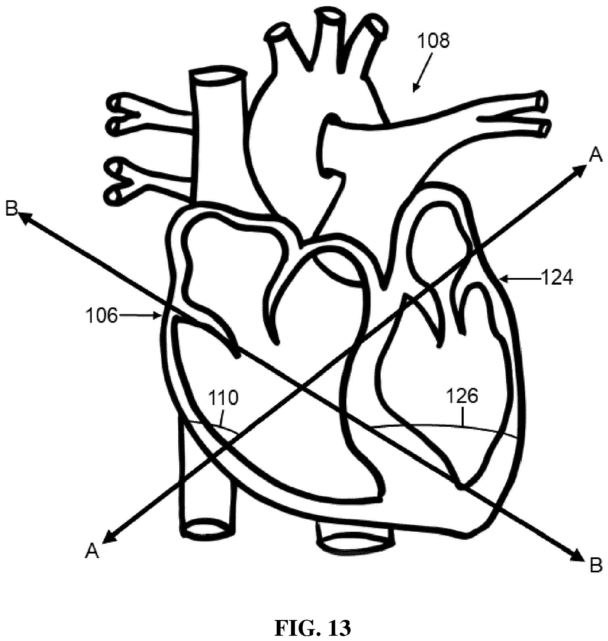

With reference to **FIG. 6****,** the main body 102 is adapted to be implanted under the skin of the patient in a first direction at a first position (depicted as line A-A in **FIG. 6** and **FIG. 13****).** In **FIG. 6****,** the first position is oriented away from a first side 106 of a heart 108 of the patient. In **FIG. 13****,** the first position is oriented towards the first side 106 of a heart 108 of the patient. The first side 106 of the heart 108 can include any side of the heart 108. The first position is at a first angle 110 from the first side 106 of the heart 108. In certain embodiments, the first angle 110 is between about 0 degrees and about 90 degrees. In another embodiment, the first angle 110 is between about 90 degrees and about 180 degrees.

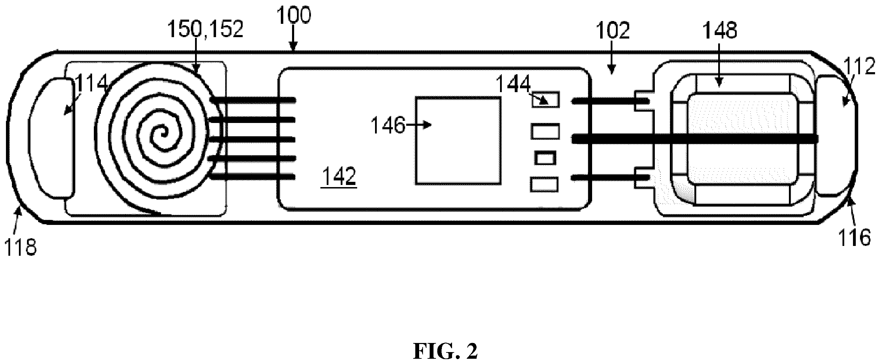

As shown in **FIG. 2****,** the main body 102 may include zero, one, or more than one, electrodes, including, but not limited to, a first electrode 112 and a second electrode 114. The main body 102 can include additional or fewer electrodes, if desired by one skilled in the art. In some embodiments, the main body 102 has no electrodes. In certain embodiments, the first electrode 112 is in electrical communication with the second electrode 114. The first electrode 112 can be disposed at a first end 116 of the main body 102 and the second electrode 114 can be disposed at a second end 118 of the main body 102. However, other locations within the main body 102 for the first electrode 112 and the second electrode 114 are possible and encompassed within the scope of the present disclosure.

Referring to **FIGS. 1-3****,** once the main body 102 is implanted under the skin of the patient in the first position, the first electrode 112 and the second electrode 114 can be adapted to receive cardiac electrical signals from the patient. The cardiac electrical signals include small electrical changes that are a consequence of cardiac muscle depolarization followed by repolarization during each cardiac cycle (heartbeat) detected relative to the first position. The first electrode 112 and the second electrode 114 can continuously or semi-continuously detect the overall magnitude of the electrical potential of the heart 108 relative to the first position. It should be appreciated that the cardiac electrical signals may be detected by other electrode combinations.

Referring to **FIG. 1****,** the main body 102 has a length 120 and a width 122. In certain embodiments, the main body is narrow, i.e., the length is greater than the width. Non-limiting examples include the length 120 being about 5 times greater than the width 122, the length 120 being about 10 times greater than the width 122, or the length 120 being about 15 times greater than the width 122. Advantageously, if the main body 102 is narrow, the incision for implanting the main body 102 under the skin of the patient can also be narrow. In certain embodiments, the length 120 is about 60 mm, the width 122 is about 9 mm, and a height of the main body 102 is about 4.2 mm. The main body 102 of the illustrated example has a rectangular shape with rounded ends. In certain embodiments, the main body 102 has a rounded shape or a cigar shape. However, one skilled in the art can select other dimensions and shapes, as desired. In certain embodiments, the main body 102 can include conductive coatings and/or insulating coatings. As a non-limiting example, the main body 102 can have a conductive coating on the first end 116 and the second end 118 of the main body 102 with insulating coatings disposed on the main body 102 between the first end 116 and the second end 118 of the main body 102. Furthermore, the one or more tail members 104 can include conductive coatings and/or insulating coatings. However, one skilled in the art may employ other coating configurations, within the scope of this disclosure.

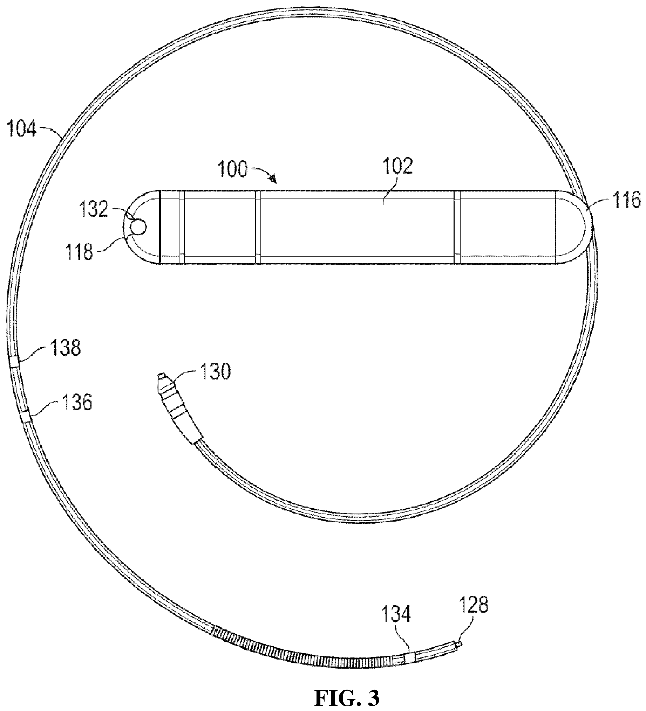

Referring to **FIG. 3** and **FIG. 6****,** the implantable cardiac monitor 100 may include the first tail member 104a. The first tail member 104a is adapted to be implanted under the skin of the patient in a second direction at a second position (shown as line B-B in **FIG. 6** and **FIG. 13****).** In **FIG. 6****,** the second position is oriented away from a second side 124 of the heart 108 of the patient. In **FIG. 13****,** the second position is oriented towards the second side 124 of the heart 108 of the patient. The second side 124 of the heart 108 can include any side of the heart 108, excluding the first side 106 of the heart 108. The second position is at a second angle 126 from the second side 124 of the heart 108. In certain embodiments, the second angle 126 is between about 0 degrees and about 90 degrees. In another embodiment, the second angle 126 is between about 90 degrees and about 180 degrees.

Referring to **FIG. 3** and **FIGS. 9****-12,** the first, second, third, and fourth tail members 104a, 104b, 104c, 104d can each be a flexible, elongate member. However, a person skilled in the art may select other structures. Referring to **FIG. 3** now for ease of description of the each of the first, second, third, and fourth tail members 104a, 104b, 104c, 104d, each tail member 104 has an end portion 128 and a connecting portion 130. The connecting portion 130 can be connected to the main body 102 of the implantable cardiac monitor 100. In some embodiments, the connecting portion 130 is removably connected to the main body 102 of the implantable cardiac monitor 100. In other embodiments, the connecting portion 130 is fixed to the main body 102 of the implantable cardiac monitor 100. As shown in **FIG. 1** and **FIG. 3****,** the main body 102 includes an aperture 132 at the second end 118 of the main body 102. Alternatively, the aperture 132 may be located at different locations on the main body 102, for example at the first end 116 of the main body 102. Regardless of its location, the aperture 132 can receive the connecting portion 130 of the tail member 104.

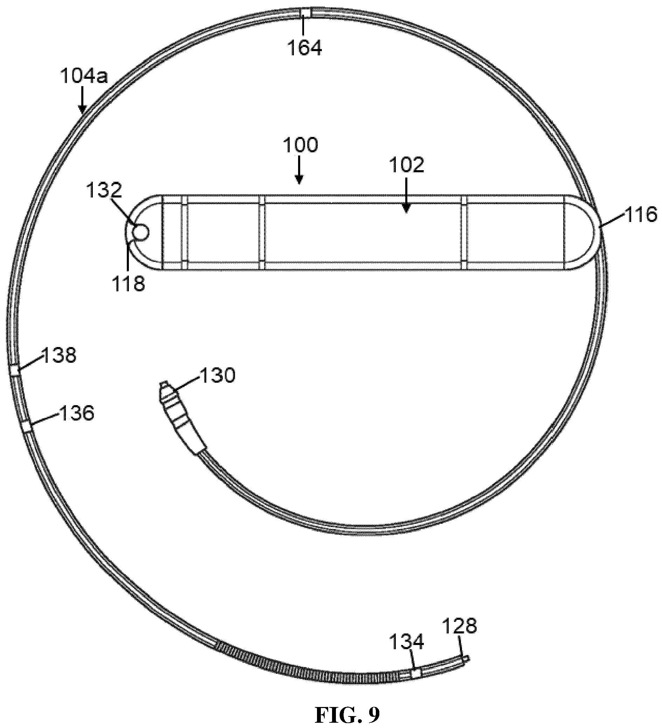

Referring still to **FIG. 3****,** each tail member 104 includes one or more electrodes. In the embodiment illustrated in **FIG. 3****,** the first tail member 104a has a first tail first electrode 134 and a first tail second electrode 136. However, the number of electrodes can be scaled if desired. As a non-limiting example, the first tail member 104a in the illustrated example in **FIG. 3** further includes a first tail third electrode 138 disposed between the connecting portion 130 and the first tail second electrode 136. In another non-limiting example, the first tail member 104a further includes a first tail fourth electrode 164 disposed between the first tail third electrode 138 and the connecting portion 130, as shown in **FIG. 9****.** In certain embodiments, the first tail first electrode 134 is in electrical communication with the first tail second electrode 136. The first tail first electrode 134 can be disposed at the end portion 128 of the first tail member 104a. The first tail second electrode 136 can be disposed between the connecting portion 130 and the first tail first electrode 134. Once the first tail member 104a is implanted under the skin of the patient in the second position, the first tail first electrode 134 and the first tail second electrode 136 are adapted to receive cardiac electrical signals from the heart of the patient. The cardiac electrical signals include small electrical changes that are a consequence of cardiac muscle depolarization followed by repolarization during each cardiac cycle (heartbeat) detected relative to the second position. The first tail first electrode 134 and the first tail second electrode 136 can continuously or semi-continuously detect the overall magnitude of the electrical potential of the heart 108 relative to the second position. It should be appreciated that the cardiac electrical signals may be detected by other electrode combinations. As a non-limiting example, one of the electrodes from the first tail member 104a may detect the cardiac electrical signals in communication with one of the electrodes 112, 114 in the main body 102. This can be particularly beneficial in situations where a certain electrode combination results in a more accurate detection of the cardiac electrical signals and avoids blind spots. In certain embodiments, the first tail member 104a has a length between about 10 mm and about 100 mm. However, other dimensions are possible and encompassed within the scope of the present disclosure.

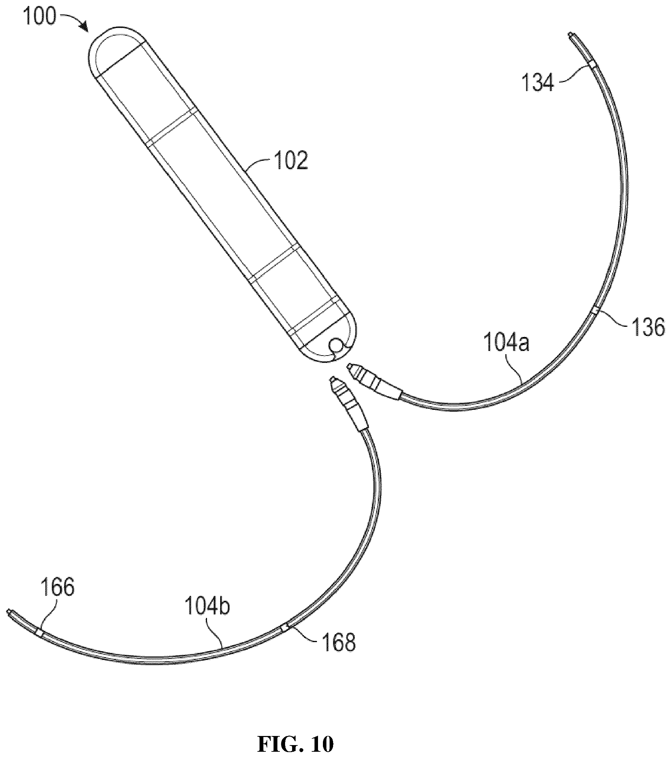

Referring now to **FIG. 10****,** the implantable cardiac monitor 100 can include the second tail member 104b. The second tail member 104b is adapted to be implanted under the skin of the patient in a third direction at a third position. The third position can be oriented from a side of the heart 108 of the patient. In certain embodiments, the third direction is different from the first direction and the second direction. In certain embodiments, the third position is different from the first position and the second position. The structural and functional aspects of the second tail member 104b can be similar, identical, or different to the first tail member 104a. Thus, the second tail member 104b can have one or more electrodes. In the embodiment illustrated in **FIG. 10****,** the second tail member 104b has a second tail first electrode 166 and a second tail second electrode 168. The one or more electrodes 166, 168 of the second tail member 104b are adapted to receive cardiac electrical signals from the heart of the patient. The cardiac electrical signals include small electrical changes that are a consequence of cardiac muscle depolarization followed by repolarization during each cardiac cycle (heartbeat) detected relative to the third position. The one or more electrodes 166, 168 of the second tail member 104b can continuously or semi-continuously detect the overall magnitude of the electrical potential of the heart 108 relative to the third position. It should be appreciated that the cardiac electrical signals may be detected by other electrode combinations. As a non-limiting example, one of the electrodes 166, 168 from the second tail member 104b may detect the cardiac electrical signals in communication with one of the electrodes 112, 114 in the main body 102, one of the electrodes 134, 136 in the first tail member 104a, or a combination thereof. This can be particularly beneficial in situations where a certain electrode combination results in a more accurate detection of the cardiac electrical signals and avoids blind spots.

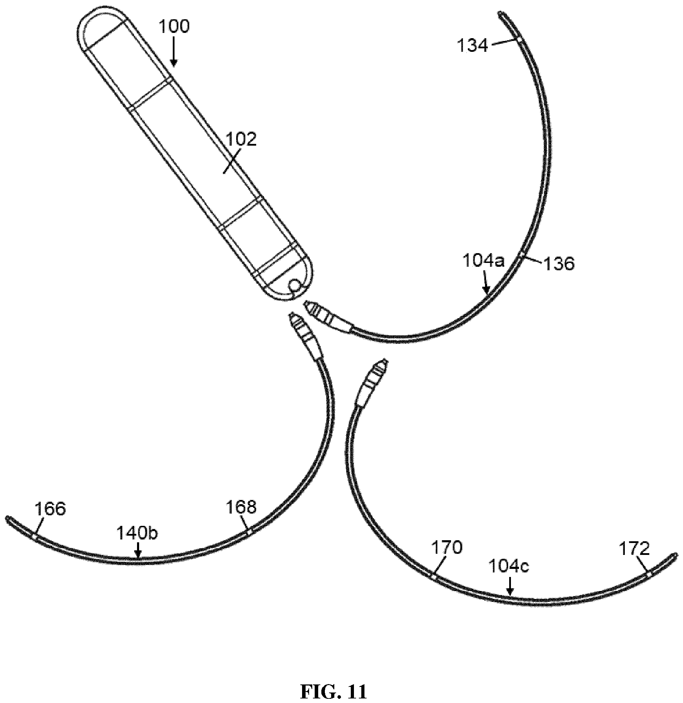

Referring now to **FIG. 11****,** the implantable cardiac monitor 100 can include the third tail member 104c. The third tail member 104c is adapted to be implanted under the skin of the patient in a fourth direction at a fourth position. The fourth position can be oriented from a side of the heart 108 of the patient. In certain embodiments, the fourth direction is different from the first direction, the second direction, and the third direction. In certain embodiments, the fourth position is different from the first position, the second position, and the third position. The structural and functional aspects of the third tail member 104c can be similar, identical, or different to the first tail member 104a. Thus, the third tail member 104c can have one or more electrodes. In the embodiment depicted in **FIG. 11****,** the third tail member 104c has a third tail first electrode 170 and a third tail second electrode 172. The one or more electrodes 170, 172 of the third tail member 104c are adapted to receive cardiac electrical signals from the heart of the patient. The cardiac electrical signals include small electrical changes that are a consequence of cardiac muscle depolarization followed by repolarization during each cardiac cycle (heartbeat) detected relative to the fourth position. The one or more electrodes 170, 172 of the third tail member 104c can continuously or semi-continuously detect the overall magnitude of the electrical potential of the heart 108 relative to the fourth position. It should be appreciated that the cardiac electrical signals may be detected by other electrode combinations. As a non-limiting example, one of the electrodes 170, 172 from the third tail member 104c may, in communication with one of the electrodes 112, 114 in the main body 102, one of the electrodes 134, 136 in the first tail member 104a, one of the electrodes 166, 168 in the second tail member 104b, or a combination thereof, detect the cardiac electrical signals. This can be particularly beneficial in situations where a certain electrode combination results in a more accurate detection of the cardiac electrical signals and avoids blind spots.

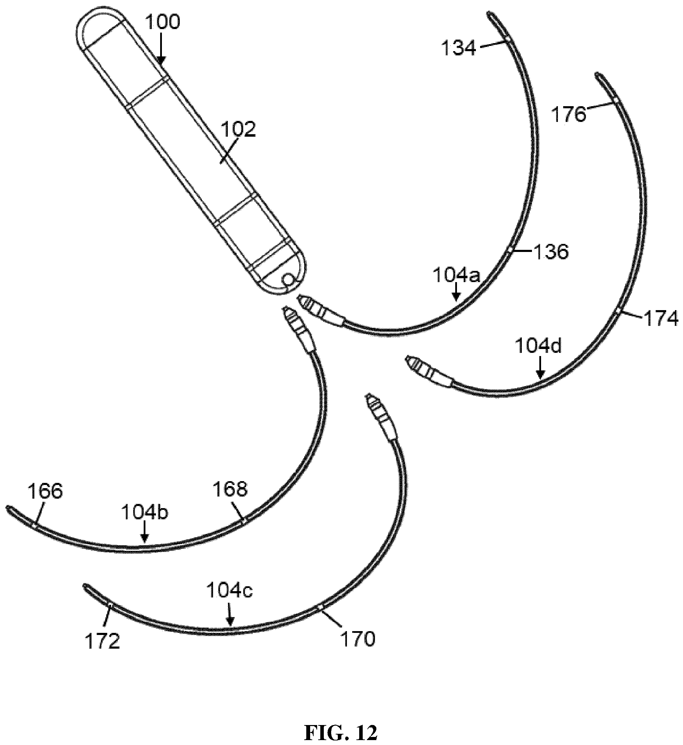

Referring now to **FIG. 12****,** the implantable cardiac monitor 100 can include the fourth tail member 104d. The fourth tail member 104d is adapted to be implanted under the skin of the patient in a fifth direction at a fifth position. The fifth position can be oriented from a side of the heart 108 of the patient. In certain embodiments, the fifth direction is different from the first direction, the second direction, the third direction, and the fourth direction. In certain embodiments, the fifth position is different from the first position, the second position, the third position, and the fourth position. The structural and functional aspects of the fourth tail member 104d can be similar, identical, or different to the first tail member 104a. Thus, the fourth tail member 104d can have one or more electrodes. In the embodiment depicted in **FIG. 12****,** the third tail member 104c has a fourth tail first electrode 174 and a fourth tail second electrode 176. The one or more electrodes 174, 176 of the fourth tail member 104d are adapted to receive cardiac electrical signals from the heart of the patient. The cardiac electrical signals include small electrical changes that are a consequence of cardiac muscle depolarization followed by repolarization during each cardiac cycle (heartbeat) detected relative to the fifth position. The one or more electrodes of the fourth tail member 104d can continuously or semi-continuously detect the overall magnitude of the electrical potential of the heart 108 relative to the fifth position. It should be appreciated that the cardiac electrical signals may be detected by other electrode combinations. As a non-limiting example, one of the electrodes 174, 176 from the fourth tail member 104d may, in communication with one of the electrodes 112, 114 in the main body 102, one of the electrodes 134, 136 in the first tail member 104a, one of the electrodes 166, 168 in the second tail member 104b, one of the electrodes 170, 172 in the third tail member 104c, or a combination thereof, detect the cardiac electrical signals. This can be particularly beneficial in situations where a certain electrode combination results in a more accurate detection of the cardiac electrical signals and avoids blind spots.

It should be appreciated that the implantable cardiac monitor 100 can include additional or fewer tail members 104, within the scope of this disclosure. In addition, in certain embodiments, the main body 102 has no electrodes and the only electrodes are present in the first tail member 104a, the second tail member 104b, the third tail member 104c, the fourth tail member 104d, or a combination thereof.

Referring now to **FIG. 2****,** the main body 102 can include a motherboard 142. The motherboard 142 can be in electrical communication with any electrodes found in the main body 102, the one or more tail members 104a, 104b, 104c, 104d, or a combination thereof. The motherboard 142 has a memory 144 and a processor 146. The memory 144 of the present examples includes a tangible, non-transitory computer readable medium with processor-executable instructions stored thereon. The processor-executable instructions include a monitoring module. The monitoring module is configured to generate monitoring data based on the cardiac electrical signals detected by any electrodes found in the main body 102, the one or more tail members 104a, 104b, 104c, 104d, or a combination thereof. More generally, such instructions may be provided to the memory (and/or from the memory to the processor) by way of a computer-readable medium. Such a computer-readable medium may include a computer-readable storage medium and/or a computer-readable transmission medium. A computer readable storage medium, which may be referred to as non-transitory, may include a memory device, magnetic storage device, optical storage device or the like. A computer-readable transmission medium may include a carrier wave, transmission signal or the like, and may occur between components of a single computer system and/or a between plural separate computer systems.

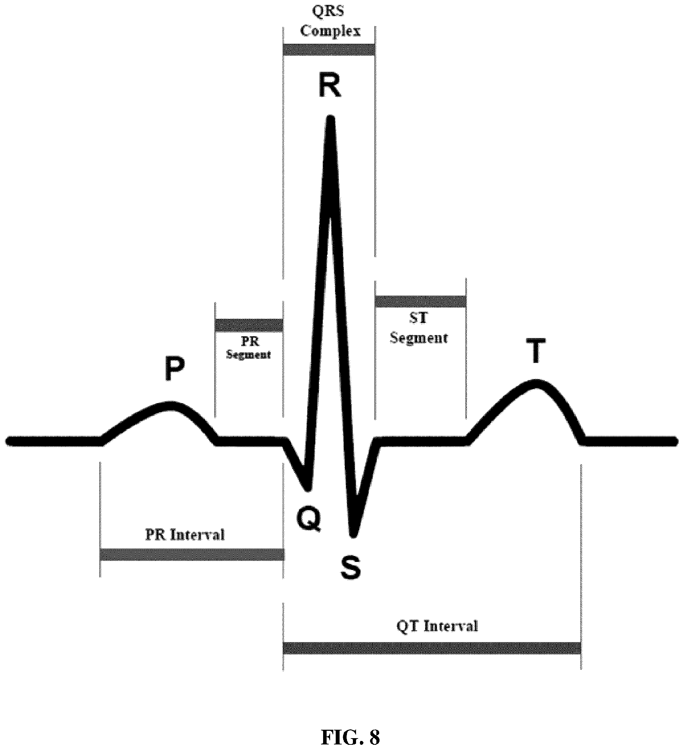

The monitoring data can include various aspects of an electrocardiogram (ECG), for example, aspects shown in **FIG. 8****.** For example, the monitoring data can include a heartrate of the patient, a p-wave, a PR interval, a PR segment, a QRS complex, a ST segment, a T wave, or combinations thereof, detected throughout the cardiac cycle. In certain embodiments, the monitoring data includes various aspects of an ECG detected relative to the first electrode 112 and the second electrode 114 in the first position (also referred to as a first channel) and various aspects of an ECG detected relative to the first tail first electrode 134 and the first tail second electrode 136 in the second position (also referred to as a second channel). Having multiple channels militates against blind spots and allows for greater accuracy and allows for the detection of a variety of cardiac events. The monitoring data can be stored in the memory 144. In certain embodiments, the monitoring data is only stored in the memory 144 based on certain parameters, for example, the current speed of the heartbeat of the patient.

The monitoring module is also configured to predict a cardiac event based on the monitoring data. Non-limiting examples of cardiac events can include atrial fibrillation, atrial flutter, ventricular fibrillation, ventricular tachycardia, pause, atrial brady, atrial tachycardia, atrial bigeminy, atrial trigeminy, ventricular bigeminy, ventricular trigeminy, acute coronary syndrome, or a combination thereof. However, it should be appreciated that other cardiac events may also be predicted. The predictions may be accomplished via wave form analysis of the monitoring data, machine learning, algorithms, or combinations thereof. In certain embodiments, the monitoring module can predict a cardiac event at least seven days before the cardiac event occurs. Advantageously, this can enable the patient to receive early warnings of certain cardiac events.

Referring now to **FIG. 2****,** the main body 102 has an internal battery 148 as a source of electric power for the implantable cardiac monitor 100. The internal battery 148 is in electrical communication with the motherboard 142. In certain embodiments, the internal battery 148 is shaped to correspond with the main body 102. In the illustrated embodiment, the internal battery 148 has a generally rectangular shape with rounded corners. However, it should be appreciated that other shapes may also be selected by one skilled in the art. In certain embodiments, the main body 102 can further include an internal coil 150. The internal coil 150 is in electrical communication with the internal battery 148. The internal coil 150 is adapted to be inductively coupled to an external coil. Advantageously, once the implantable cardiac monitor 100 is implanted under the skin of the patient, the external coil can be placed above the skin of the patient and in proximity to the internal coil 150 to inductively couple the external coil to the internal coil 150 to enable wireless charging of the internal battery 148. However, other methods of charging the internal battery 148 are possible and encompassed within the scope of the present disclosure.

Referring still to **FIG. 2****,** the implantable cardiac monitor 100 can also include an internal antenna 152. The internal antenna 152 is in electrical communication with the motherboard 142. In the embodiment illustrated in **FIG. 2****,** the internal antenna 152 is integrated with the internal coil 150. However, the internal antenna 152 can be separate from the internal coil 150, and such embodiments are within the scope of the present disclosure. The internal antenna 152 is configured to be placed in wireless communication with an external monitoring device. Non-limiting examples of external monitoring devices include mobile devices, such as phones, smart watches, tablets, laptops, or other personal computers. The internal antenna 152 enables the monitoring data to be transferred from the implantable cardiac monitor 100 to the external monitoring device. A non-limiting example of the internal antenna 152 includes a Bluetooth^{®} antenna. However, other short-range or long-range antennas, such as cellular antennas, may be utilized for the internal antenna 152.

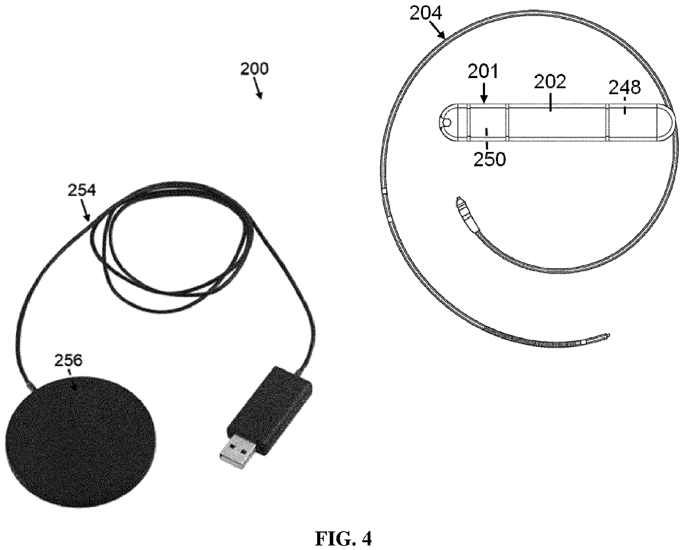

**FIG. 4** shows an example cardiac monitoring system 200. The cardiac monitoring system 200 includes an implantable cardiac monitor 201 and an external charging device 254. The implantable cardiac monitor 201 can be similar or identical to the implantable cardiac monitor 100, except as described below. In this embodiment, the implantable cardiac monitor 201 has a main body 202 and one or more tail members 204. The main body 202 includes an internal battery 248 and an internal coil 250. The external charging device 254 includes an external coil 256. Once the implantable cardiac monitor 201 is implanted under the skin of the patient, the external charging device 254 can be placed above the skin on the patient in close proximity to the implantable cardiac monitor 201. Once in close proximity, the external coil 256 can be inductively coupled with the internal coil 250 in the main body 202 of the implantable cardiac monitor 201 to enable wireless charging the internal battery 248. Advantageously, this allows the internal battery 248 of the implantable cardiac monitor 201 to be charged without requiring a physical connection between a recharging device and the internal battery 248. Since the internal battery 248 can be easily recharged with the external charging device 254, the implantable cardiac monitor 201 can remain in the patient for a longer duration. A non-limiting example of the external charging device 254 can include a patch having the external coil 256. However, one skilled in the art can change the structure of the external charging device 254, as desired.

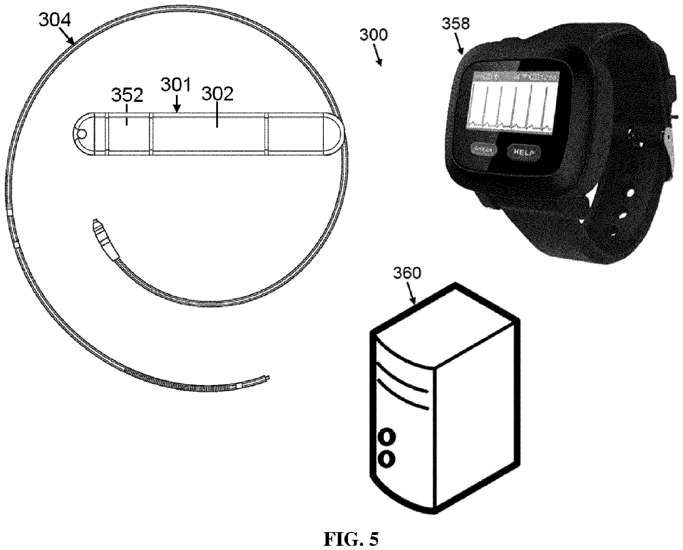

**FIG. 5** shows another example cardiac monitoring system 300. The cardiac monitoring system 300 includes an implantable cardiac monitor 301 and an external monitoring device 358. The implantable cardiac monitor 301 can be similar or identical to the implantable cardiac monitor 100, except as described below. In this embodiment, the implantable cardiac monitor 301 has a main body 302 and one or more tail members 304. The main body 302 includes an internal antenna 352. The external monitoring device 358 is configured to be in wireless communication with the internal antenna 352 of the implantable cardiac monitor 301. The external monitoring device 358 can include Bluetooth^{®} communication capabilities, and cellular communication capabilities, for example 4G LTE. The wireless communication enables the implantable cardiac monitor 301 to transfer the monitoring data to the external monitoring device 358. The implantable cardiac monitor 301 can be configured to continuously or semi-continuously transfer monitoring data. In certain embodiments, the patient can initiate a transfer of the monitoring data using the external monitoring device 358. Non-limiting examples of the external monitoring device 358 include mobile devices, such as phones, smart watches, tablets, laptops, smart necklaces, or other personal computers.

In certain embodiments, the external monitoring device 358 is configured to provide an alert to the patient based on the monitoring data. For example, if the monitoring module has predicted a cardiac event is occurring or may soon occur, the external monitoring device 358 can alert the user that the cardiac event is occurring or is likely to occur. Non-limiting examples of the alert include a visual alert, an audio alert, a haptic feedback alert, or combinations thereof. A non-limiting example of a visual alert includes a text message. A non-limiting example of the audio alert includes a ring noise. A non-limiting example of the haptic feedback includes vibration via a small motor located in the external monitoring device 358. One skilled in the art can employ other methods and technologies for the alert, within the scope of the disclosure.

The external monitoring device 358 can also be configured to permit user customization. For example, the external monitoring device 358 can allow the patient to disable or enable reminders or alarms. In certain embodiments, the external monitoring device 358 can be configured to display certain aspects of the monitoring data to the patient. Other features and options may also be included for the external monitoring device 358, within the scope of the present disclosure.

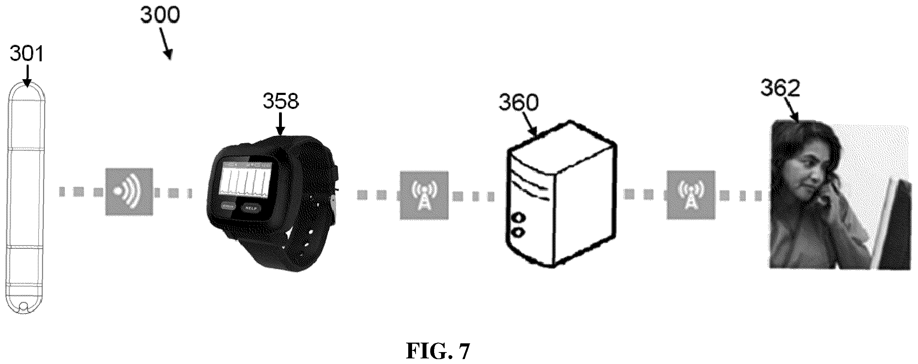

Referring still to **FIG. 5****,** the cardiac monitoring system 300 can include a server 360. The number of servers 360 is not particularly limited. For example, more than one server 360 may be included in the cardiac monitoring system 300. In addition, the server 360 can include a cloud network. The server 360 can be in wireless communication with the implantable cardiac monitor 301, the external monitoring device 358, or a combination thereof. The server 360 is configured to receive the monitoring data via the wireless communication. As shown in **FIG. 7****,** the implantable cardiac monitor 301 can be in short-range wireless communication with the external monitoring device 358 and the external monitoring device 358 can be in long-range wireless communication, e.g., cellular communication, with the server 360. However, different communication methods, protocols, and technologies can be employed to enable the transfer of the monitoring data to the server 360, and the use of such other methods, protocols, and technologies is encompassed within the scope of the present disclosure.

Referring now to **FIG. 7****,** the server 360 can be configured to provide monitoring data or the alert to medical service providers 362. For example, if the monitoring module has predicted a cardiac event is occurring or may soon occur, the server 360 can contact a hospital to arrange an ambulance to come for the patient. In another example, the server 360 can transfer the monitoring data to the medical service providers 362 for review and analysis.

A method of performing a medical procedure can include advancing the main body of the implantable cardiac monitor under the skin of the patient and advancing the tail member of the implantable cardiac monitor under the skin of the patient. The main body extends into the first direction at the first position and the tail member extends into the second direction at the second position. The first position is oriented away from the first side of the heart and the second position is oriented away from the second side of the heart. The first position is at a first angle from the first side of the heart. In certain embodiments, the first angle is between from about 0 degrees and about 180 degrees. The second position is at a second angle from the second side of the heart. In certain embodiments, the second angle is between from about 0 degrees and about 180 degrees.

In certain embodiments, the method further includes a step of advancing the second tail member of the implantable cardiac monitor under the skin of the patient at the third position and extending into the third direction. In certain embodiments, the method further includes a step of advancing the third tail member of the implantable cardiac monitor under the skin of the patient at the fourth position and extending into the fourth direction. In certain embodiments, the method further includes a step of advancing the fourth tail member of the implantable cardiac monitor under the skin of the patient at the fifth position and extending into the fifth direction.

In certain embodiments, the main body and the tail member are inserted between about 2 mm and about 15 mm under the skin of the patient and may be less than about 15 mm from the heart of the patient. However, it should be appreciated that one skilled in the art can insert the main body and the tail member at different locations, as desired. For example, in certain embodiments, the main body and the tail member may be disposed more than 15 mm under the skin of the patient.

Advantageously, the implantable cardiac monitor can be implanted at a physician's office or medical center without requiring the patient to be put under general anesthesia. The procedure can include creating a minor incision in the skin of the patient. In the embodiments where the main body is narrow, the incision for the implant can be narrow.

A method of monitoring the cardiac cycle of a patient can include detecting cardiac electrical signals by the implantable cardiac monitor, generating the monitoring data based on detected electrical signals, storing the monitoring data into the memory of the implantable cardiac monitor, transferring the monitoring data from the implantable cardiac monitor to the external monitoring device, and contacting, through the server, medical service providers based on the monitoring data.

Advantageously, the implantable cardiac monitor, systems, and associated methods described herein facilitate detecting certain cardiac events, including, but not limited to, atrial fibrillation, atrial flutter, ventricular fibrillation, ventricular tachycardia, pause, atrial brady, atrial tachycardia, atrial bigeminy, atrial trigeminy, ventricular bigeminy, ventricular trigeminy, acute coronary syndrome, or a combination thereof. In addition, the embodiments of the implantable cardiac monitors which feature the internal coil allow for the device to be wirelessly charged, which allows the implantable cardiac monitor to stay in the body of a patient for longer durations.

Therefore, from on perspective there have been described approaches for an implantable cardiac monitor and associated methods. The implantable cardiac monitor comprises a main body having a first electrode and a second electrode, the first electrode being in electrical communication with the second electrode, and the first electrode and the second electrode being adapted to receive cardiac electrical signals from a patient relative to a first position; and a tail member connected to the main body and having a third electrode and a fourth electrode, the third electrode being in electrical communication with the fourth electrode, and the third electrode and the fourth electrode being adapted to receive cardiac electrical signals from the patient relative to a second position.

Further examples are set out in the following numbered clauses.

Clause 1. An implantable cardiac monitor comprising: a main body having a first electrode and a second electrode, the first electrode being in electrical communication with the second electrode, and the first electrode and the second electrode being adapted to receive electrical signals relative to a first position; and a tail member connected to the main body and having a third electrode and a fourth electrode, the third electrode being in electrical communication with the fourth electrode, and the third electrode and the fourth electrode being adapted to receive electrical signals relative to a second position.

Clause 2. The implantable cardiac monitor of clause 1, wherein the main body further comprises a motherboard including a processor and a memory, the memory including a computer readable medium comprising processor-executable instructions.

Clause 3. The implantable cardiac monitor of clause 2, wherein the motherboard includes a monitoring module configured to generate monitoring data based on received cardiac electrical signals from a patient, and the memory is configured to store the monitoring data.

Clause 4. The implantable cardiac monitor of clause 3, wherein the monitoring data includes at least one of a heartrate, a P-wave, a PR interval, a PR segment, a QRS complex, a ST segment, a T wave, or a combination thereof.

Clause 5. The implantable cardiac monitor of clause 3 or 4, wherein the monitoring module is configured to predict a cardiac event based on the monitoring data.

Clause 6. The implantable cardiac monitor of clause 5, wherein the cardiac event includes at least one of atrial fibrillation, atrial flutter, ventricular fibrillation, ventricular tachycardia, pause, atrial brady, atrial tachycardia, atrial bigeminy, atrial trigeminy, ventricular bigeminy, ventricular trigeminy, acute coronary syndrome, or a combination thereof.

Clause 7. The implantable cardiac monitor of clause 5 or 6, wherein the monitoring module includes an algorithm configured to predict the cardiac event.

Clause 8. The implantable cardiac monitor of any of clauses 2 to 7, wherein the main body further comprises an internal battery in electrical communication with the motherboard.

Clause 9. The implantable cardiac monitor of clause 8, wherein the internal battery has a rectangular shape with rounded corners.

Clause 10. The implantable cardiac monitor of clause 8 or 9, wherein the main body further comprises an internal coil in electrical communication with the internal battery and configured to be inductively coupled to an external coil.

Clause 11. The implantable cardiac monitor of any of clauses 2 to 10, wherein the main body further comprises an internal antenna in electrical communication with the motherboard and configured to be in wireless communication with an external monitoring device.

Clause 12. The implantable cardiac monitor of any preceding clause, further comprising a second tail member having one or more electrodes being adapted to receive electrical signals relative to a third position.

Clause 13. The implantable cardiac monitor of clause 12, further comprising a third tail member having one or more electrodes being adapted to receive electrical signals relative to a fourth position.

Clause 14. The implantable cardiac monitor of clause 13, further comprising a fourth tail member having one or more electrodes being adapted to receive electrical signals relative to a fifth position.

Clause 15. A cardiac monitoring system comprising: an implantable cardiac monitor comprising a main body having a first electrode, a second electrode, and an internal coil, the first electrode being in electrical communication with the second electrode, and the first electrode and the second electrode being adapted to receive electrical signals relative to a first position; a tail member connected to the main body and having a third electrode and a fourth electrode, the third electrode being in electrical communication with the fourth electrode, and the third electrode and the fourth electrode being adapted to receive electrical signals relative to a second position; and an external charging device having an external coil configured to be inductively coupled to the internal coil.

Clause 16. A cardiac monitoring system comprising: an implantable cardiac monitor comprising a main body having a first electrode, a second electrode, and an internal antenna, the first electrode being in electrical communication with the second electrode, and the first electrode and the second electrode being adapted to receive electrical signals relative to a first position; a tail member connected to the main body and having a third electrode and a fourth electrode, the third electrode being in electrical communication with the fourth electrode, and the third electrode and the fourth electrode being adapted to receive electrical signals relative to a second position; and an external monitoring device configured to be in communication with the internal antenna and receiving monitoring data based on received electrical signals relative to the first position and relative to the second position.

Clause 17. The cardiac monitoring system of clause 16, wherein the external monitoring device is a smart watch.

Clause 18. The cardiac monitoring system of clause 16 or 17, wherein the external monitoring device is configured to provide an alert based on the monitoring data.

Clause 19. The cardiac monitoring system of clause 16, 17 or 18, further comprising a server configured to be in communication with at least one of the implantable cardiac monitor or the external monitoring device and receive the monitoring data.

Clause 20. The cardiac monitoring system of clause 19, wherein the server is configured to provide an alert or the monitoring data to medical service providers.

Clause 21. A method of monitoring the heart of a patient, the method comprising: detecting cardiac electrical signals with an implantable cardiac monitor; generating monitoring data based on the detected cardiac electrical signals; storing the monitoring data into a memory of the implantable cardiac monitor; transferring the monitoring data from the implantable cardiac monitor to an external monitoring device; transferring the monitoring data from the external monitoring device to a server; and contacting, through the server, medical service providers based on the monitoring data.

Clause 22. An implantable cardiac monitor comprising: a main body having a motherboard including a processor and a memory, the memory including a computer readable medium comprising processor-executable instructions; and a tail member connected to the main body and having one or more electrodes being adapted to receive electrical signals relative to a first position.

Clause 23. The implantable cardiac monitor of clause 22, wherein the motherboard includes a monitoring module configured to generate monitoring data based on received cardiac electrical signals from a patient, and the memory is configured to store the monitoring data.

Clause 24. The implantable cardiac monitor of clause 23, wherein the monitoring data includes at least one of a heartrate, a P-wave, a PR interval, a PR segment, a QRS complex, a ST segment, a T wave, or a combination thereof.

Clause 25. The implantable cardiac monitor of clause 23 or 24, wherein the monitoring module is configured to predict a cardiac event based on the monitoring data.

Clause 26. The implantable cardiac monitor of clause 25, wherein the cardiac event includes at least one of atrial fibrillation, atrial flutter, ventricular fibrillation, ventricular tachycardia, pause, atrial brady, atrial tachycardia, atrial bigeminy, atrial trigeminy, ventricular bigeminy, ventricular trigeminy, acute coronary syndrome, or a combination thereof.

Clause 27. The implantable cardiac monitor of clause 25 or 26, wherein the monitoring module includes an algorithm configured to predict the cardiac event.

Clause 28. The implantable cardiac monitor of any of clauses 22 to 27, wherein the main body further comprises an internal battery in electrical communication with the motherboard.

Clause 29. The implantable cardiac monitor of clause 28, wherein the internal battery has a rectangular shape with rounded corners.

Clause 30. The implantable cardiac monitor of clause 28 or 29, wherein the main body further comprises an internal coil in electrical communication with the internal battery and configured to be inductively coupled to an external coil.

Clause 31. The implantable cardiac monitor of any of clauses 22 to 30, wherein the main body further comprises an internal antenna in electrical communication with the motherboard and configured to be in wireless communication with an external monitoring device.

Clause 32. The implantable cardiac monitor of any of clauses 22 to 31, further comprising a second tail member having one or more electrodes being adapted to receive electrical signals relative to a second position.

Clause 33. The implantable cardiac monitor of clause 32, further comprising a third tail member having one or more electrodes being adapted to receive electrical signals relative to a third position.

Clause 34. The implantable cardiac monitor of clause 33, further comprising a fourth tail member having one or more electrodes being adapted to receive electrical signals relative to a fourth position.

Clause 35. The implantable cardiac monitor of clause 34, wherein the main body includes one or more electrodes being adapted to receive electrical signals relative to a fifth position.

Clause 36. The implantable cardiac monitor of any of clauses 22 to 35, wherein the main body includes one or more electrodes being adapted to receive electrical signals relative to a second position.

Clause 37. A method of performing a medical procedure, the method comprising: advancing a main body of an implantable cardiac monitor under the skin of a patient; and advancing a tail member of the implantable cardiac monitor under the skin of the patient; wherein the main body extends into a first position and in first direction, the tail member extends into a second position and in a second direction, the first position being oriented away from one side of a heart of the patient, and the second position being oriented away from another side of the heart of the patient.

Clause 38. The method of clause 37, further including a step of advancing a second tail member into a third position and extending the second tail member in a third direction.

Clause 39. The method of clause 38, further including a step of advancing a third tail member into a fourth position and extending the third tail member in a fourth direction.

Clause 40. The method of clause 39, further including a step of advancing a fourth tail member into a fifth position and extending the fourth tail member in a fifth direction.

The principle and mode of operation of this disclosure have been explained and illustrated in its various embodiments. However, it must be understood that this disclosure may be practiced otherwise than as specifically explained and illustrated without departing from its spirit or scope.

## Claims

1. An implantable cardiac monitor comprising:
a main body having a first electrode and a second electrode, the first electrode being in electrical communication with the second electrode, and the first electrode and the second electrode being adapted to receive electrical signals relative to a first position; and
a tail member connected to the main body and having a third electrode and a fourth electrode, the third electrode being in electrical communication with the fourth electrode, and the third electrode and the fourth electrode being adapted to receive electrical signals relative to a second position.

2. The implantable cardiac monitor of claim 1, wherein the main body further comprises a motherboard including a processor and a memory, the memory including a computer readable medium comprising processor-executable instructions.

3. The implantable cardiac monitor of claim 2, wherein the motherboard includes a monitoring module configured to generate monitoring data based on received cardiac electrical signals from a patient, and the memory is configured to store the monitoring data, for example wherein the monitoring data includes at least one of a heartrate, a P-wave, a PR interval, a PR segment, a QRS complex, a ST segment, a T wave, or a combination thereof.

4. The implantable cardiac monitor of claim 3, wherein the monitoring module is configured to predict a cardiac event based on the monitoring data, for example wherein the cardiac event includes at least one of atrial fibrillation, atrial flutter, ventricular fibrillation, ventricular tachycardia, pause, atrial brady, atrial tachycardia, atrial bigeminy, atrial trigeminy, ventricular bigeminy, ventricular trigeminy, acute coronary syndrome, or a combination thereof.

5. The implantable cardiac monitor of claim 4, wherein the monitoring module includes an algorithm configured to predict the cardiac event.

6. The implantable cardiac monitor of any of claims 2 to 5, wherein the main body further comprises an internal battery in electrical communication with the motherboard, for example wherein the internal battery has a rectangular shape with rounded corners.

7. The implantable cardiac monitor of claim 6, wherein the main body further comprises an internal coil in electrical communication with the internal battery and configured to be inductively coupled to an external coil.

8. The implantable cardiac monitor of any of claims 2 to 7, wherein the main body further comprises an internal antenna in electrical communication with the motherboard and configured to be in wireless communication with an external monitoring device.

9. The implantable cardiac monitor of any preceding claim, further comprising a second tail member having one or more electrodes being adapted to receive electrical signals relative to a third position, for example further comprising a third tail member having one or more electrodes being adapted to receive electrical signals relative to a fourth position, and for example further comprising a fourth tail member having one or more electrodes being adapted to receive electrical signals relative to a fifth position.

10. A cardiac monitoring system comprising:
the implantable cardiac monitor of any preceding claim, wherein the main body has an internal coil;
an external charging device having an external coil configured to be inductively coupled to the internal coil.

11. A cardiac monitoring system comprising:
the implantable cardiac monitor of any preceding claims, wherein the main body has an internal antenna;
an external monitoring device configured to be in communication with the internal antenna and receiving monitoring data based on received electrical signals relative to the first position and relative to the second position.

12. The cardiac monitoring system of claim 11, wherein the external monitoring device is a smart watch and/or is configured to provide an alert based on the monitoring data.

13. The cardiac monitoring system of claim 11 or 12, further comprising a server configured to be in communication with at least one of the implantable cardiac monitor or the external monitoring device and receive the monitoring data, for example wherein the server is configured to provide an alert or the monitoring data to medical service providers.

14. An implantable cardiac monitor comprising:
a main body having a motherboard including a processor and a memory, the memory including a computer readable medium comprising processor-executable instructions; and
a tail member connected to the main body and having one or more electrodes being adapted to receive electrical signals relative to a first position.

15. The implantable cardiac monitor of claim 14, wherein the motherboard includes a monitoring module configured to generate monitoring data based on received cardiac electrical signals from a patient, and the memory is configured to store the monitoring data.

16. The implantable cardiac monitor of claim 15, wherein the monitoring data includes at least one of a heartrate, a P-wave, a PR interval, a PR segment, a QRS complex, a ST segment, a T wave, or a combination thereof.

17. The implantable cardiac monitor of claim 15 or 16, wherein the monitoring module is configured to predict a cardiac event based on the monitoring data, for example wherein the cardiac event includes at least one of atrial fibrillation, atrial flutter, ventricular fibrillation, ventricular tachycardia, pause, atrial brady, atrial tachycardia, atrial bigeminy, atrial trigeminy, ventricular bigeminy, ventricular trigeminy, acute coronary syndrome, or a combination thereof, and for example wherein the monitoring module includes an algorithm configured to predict the cardiac event.

18. The implantable cardiac monitor of any of claims 14 to 17, wherein the main body further comprises an internal battery in electrical communication with the motherboard, for example wherein the internal battery has a rectangular shape with rounded corners.

19. The implantable cardiac monitor of claim 18, wherein the main body further comprises an internal coil in electrical communication with the internal battery and configured to be inductively coupled to an external coil.

20. The implantable cardiac monitor of any of claims 14 to 19, wherein the main body further comprises an internal antenna in electrical communication with the motherboard and configured to be in wireless communication with an external monitoring device.

21. The implantable cardiac monitor of any of claims 14 to 20, further comprising a second tail member having one or more electrodes being adapted to receive electrical signals relative to a second position.

22. The implantable cardiac monitor of claim 21, further comprising a third tail member having one or more electrodes being adapted to receive electrical signals relative to a third position, for example further comprising a fourth tail member having one or more electrodes being adapted to receive electrical signals relative to a fourth position; and for example wherein the main body includes one or more electrodes being adapted to receive electrical signals relative to a fifth position.

23. The implantable cardiac monitor of any of claims 14 to 22, wherein the main body includes one or more electrodes being adapted to receive electrical signals relative to a second position.
